(19) Europäisches Patentamt
European Patent Office
Office européen des brevets

(11) **EP 3 836 828 B1**

(12) **EUROPEAN PATENT SPECIFICATION**

(45) Date of publication and mention
of the grant of the patent:
**12.06.2024 Bulletin 2024/24**

(21) Application number: **18773615.2**

(22) Date of filing: **18.08.2018**

(51) International Patent Classification (IPC):
***A61B 5/00*** *(2006.01)*    ***A61B 5/024*** *(2006.01)*

(52) Cooperative Patent Classification (CPC):
**A61B 5/6802; A61B 5/7285;** A61B 5/0006;
A61B 5/024; A61B 5/318; A61B 2562/0219

(86) International application number:
**PCT/IB2018/056260**

(87) International publication number:
**WO 2020/039226 (27.02.2020 Gazette 2020/09)**

(54) **METHOD FOR SYNCHRONIZATION OF A MULTITUDE OF WEARABLE DEVICES**

VERFAHREN ZUR SYNCHRONISATION EINER VIELZAHL VON TRAGBAREN VORRICHTUNGEN

PROCÉDÉ DE SYNCHRONISATION D'UNE MULTITUDE DE DISPOSITIFS PORTABLES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**23.06.2021 Bulletin 2021/25**

(73) Proprietor: **SmartCardia SA
1015 Lausanne (CH)**

(72) Inventors:
• **MURALI, Srinivasan
1012 Lausanne (CH)**

• **RINCON VALLEJOS, Francisco Javier
1020 Renens (CH)**

(74) Representative: **Linhart, Friedrich Karl Eberhard
La Couronnette
1166 Perroy (CH)**

(56) References cited:
WO-A1-2016/110804    WO-A1-2018/013569
US-A1- 2005 273 017    US-A1- 2011 160 601
US-A1- 2012 123 701    US-A1- 2013 237 775
US-A1- 2014 275 888    US-A1- 2016 066 808

## Description

## Technical Field

The invention relates to a method for synchronization of a multitude of wearable devices according to claim 1.

## Background Art

For accurate monitoring of several conditions of living organisms, such as for example Atrial Fibrillation (AF), a multiple lead electrocardiogram (abbreviated as ECG) monitoring is needed, so that if there is noise in one of the leads, another lead can be used. In traditional systems, the different lead wires are connected to different parts of the body of the living organism on one end, and the wires are connected to a single electronic unit on their other ends. Thus, all the different ECG lead signals are time synchronized or can be easily synchronized because they are all interconnected with each other and with the electronic units by means of cables.

For other health conditions, multiple bio-signals (such as one or more lead ECG, pulse from finger) are measured and monitored. In traditional systems, the different measurement units are connected to an electronic unit by means of cables, and the time synchronization between them can also be performed easily by the electronic unit.

Recently, as an alternative to cable-based ECG and bio-sensing systems, wearable devices have been presented. Such wearable devices can for example take the form of wearable patches that comprise an electronic circuitry comprising for example a processor, one or more sensors and a transmitter. Furthermore, such wearable patches typically comprise an adhesive surface by means of which they can be attached to a body. When using such wearable devices and in particular wearable patches, given the small size of such devices, it is possible to use multiple wearable devices and/or patches at different parts of the body to obtain multi lead ECG signals and measure other bio-signals, such as the pulse, blood pressure, phonocardiogram or impedance cardiogram. A single patch at a location could also measure more than one bio-signal simultaneously.

However, because the wearable devices are not interconnected by means of cables, it is not easy to properly time synchronize the different wearable devices. Such a time synchronization is, however, important in order to accurately align the data (such as for example ECG signals) obtained from the different patches temporally (that is, for example on a common time axis).

Furthermore, time differences of body signals measured at different parts of the body by means of wearable devices such as the previously mentioned wearable patches can in principle be used to acquire health information concerning the body. However, for such health information to be accurate, the different wearable devices should be time synchronized as precisely as possible.

WO 2018/013569 A1 discloses a multi-sensor blood pressure measurement system. US 2014/275888 A1 discloses a wearable wireless multisensor health monitor with head photoplethysmography.

## Problem to be Solved

It is the object of the invention to solve or to at least diminish the above-mentioned disadvantages. In particular, it is the object of the invention to find ways to enable and/or to improve the time synchronization of wearable devices, for example wearable patches, which are being employed for measuring body signals.

## Solution to the Problem

This problem is solved by a method for synchronization of a multitude of wearable devices according to claim 1.

In the context of this application, the term "synchronization" refers to a time-wise synchronization. The expression "wearable device" is to be understood as any electronic device that is configured to be at least temporarily attached to a body, for example a wearable patch as previously described or a watch or smartwatch, or a wrist band, a chest band or a collar. The term "multitude" is to be understood in the sense of "at least two". A "body of a living organism" is typically a human body or an animal body. A "software application" is to be understood as any kind of computer program, for example a software tool running on a desktop computer or laptop, or yet an application running on a smartphone, mobile phone, tablet, smartwatch or any other portable electronic device, or yet a cloud-based application. The term "coordination device" is to be understood such that it relates to any kind of electronic device that is able to run a software application in the sense of the present disclosure. It is not excluded that the coordination device is at the same time a wearable device in the sense of the application. For example, a smartwatch can theoretically at the same time be part of the multitude of wearable devices and serve as coordination device. The term "application time information" typically refers to an absolute time, such as "12:30:44", i.e. 12 h 30 m 44 s, but can in principle also refer to a relative time, for example the current value of a counter which has been started at a particular moment in time.

The fact that there is one coordination device with a software application running on it and that this software application sends an application time information to all wearable devices is an effective way to enable synchronization of the wearable devices.

In typical embodiments, at least one of the wearable devices is a wearable patch for monitoring at least one body signal, in particular a pulse signal and/or an ECG signal, of the body. In the context of this application, the term "wearable patch" refers to a device that comprises an electronic circuitry, wherein the electronic circuitry typically comprises a processor and/or a sensor, typically a pulse sensor, and/or a transmitter. Furthermore, such wearable patches typically comprise an adhesive surface by means of which they can be attached to a body. The use of wearable patches is advantageous because they are typically especially designed for measuring body signals such as ECG signals or pulse signals. In addition to that, such patches are typically comparably small and/or flat and can be attached to various parts of the body, e.g. on chest, stomach, arm or leg of human being or animal. However, it is not mandatory to use wearable patches: it is also possible to use for example a combination of a smartwatch and a chest band as wearable devices. In typical embodiments, all wearable devices are wearable patches. This has the advantage that synchronization can be particularly straightforward because there is only one device type. In other typical embodiments, one wearable device is a smartwatch and the other wearable devices are wearable patches.

In typical embodiments, the coordination device is a mobile phone and/or a tablet computer and/or a laptop, and/or a computer and/or a Bluetooth hub and/or a router and/or any type of hardware device, preferably any type of portable electronic device. The use of such devices as coordination device has the advantage that these devices are widely available and can all easily be configured to facilitate the execution of the software application. In a particular embodiment, the coordination device is a system with distributed components, for example a combination of a smartwatch, a smartphone and a remote computer. In a particular embodiment, the coordination device comprises a smartwatch or is a smartwatch. In a particular embodiment, the coordination device is at the same time a wearable device in the sense of this disclosure.

In typical embodiments, the application time information is sent to the wearable devices via a wireless network, preferably using a wireless protocol such as Bluetooth and/or Zigbee and/or WiFi and/or GSM. The use of such networks is advantageous for example because they are standard networks that are widely available and reliable and have well-defined characteristics. However, it is in principle also possible that the time information is sent through point-to-point connections and/or using a custom protocol.

In typical embodiments, the application time information corresponds to an internal time of the coordination device, wherein the internal time is preferably a network time, in particular a time provided by a mobile carrier network, or a time set by the user. Choosing one of these times as application time information is advantageous, because these times are typically easily available and easy to understand. However, the application time information can in principle also correspond to a more abstract value, such as the value of an internal counter of the coordination device.

Each wearable device stores the application time information, preferably in a wearable device processor comprised in each wearable device, and starts a counter for maintaining a wearable device time in line with the application time information, preferably in line with the internal time of the coordination device. In the case where a wearable device is actually a wearable patch, the wearable device processor is referred to as patch processor and the wearable device time is referred to as patch time. Starting an internal counter in each device based on the application time information received from the coordination device is advantageous because once the application time information has been received by the different wearable devices, each device can in principle maintain its proper wearable device time, which is in line - and thus synchronized - with wearable device times of any of the other wearable devices. Like this, no further interaction with the coordination device is in principle necessary anymore, and it is for example possible to at least temporarily switch of the coordination device or cut off a connection between the multitude of wearable devices and the coordination device. Furthermore, it is possible to then change the coordination device. However, counters in the wearable devices are not absolutely mandatory. It is for example also possible for the coordination device to periodically send application time information to the wearable devices.

In each particular wearable device, the counter of that particular wearable device uses a sampling rate of at least one sensor comprised in that particular wearable device for counting, wherein the sensor is preferably a pulse sensor. As a matter of fact, sensors often comprise oscillating crystals for maintaining sampling rates of the sensor, and the sampling rates created by these oscillating crystals can therefore advantageously used as counters of the wearable devices. Put differently, in particular embodiments, an oscillating crystal comprised in a sensor of the wearable device forms part or is the counter. This has the advantage of reducing the number of components in the wearable devices. It is of course also possible that each particular wearable device comprises a separate counter, for example one that comprises a distinct oscillating crystal, or that only some or one of the wearable devices use a sampling rate of at least one sensor comprised in that particular wearable device for counting.

In typical embodiments, the multitude of wearable devices comprises at least two, preferably at least three, more preferably at least five wearable devices, wherein the wearable devices are preferably essentially identical. The expression "essentially identical" is to be understood such that the wearable devices are in principle able to all fulfill the same functions but that it is not excluded that they for example slightly differ in size or form. In typical embodiments, all wearable devices are identical. In other embodiments, some of the wearable devices are identical and others are not. For example, it is possible that the multitude of wearable devices comprises two identical wearable patches placed at different parts of a body, e.g. on the chest and on the stomach, and a smartwatch placed at a wrist of the body. Essentially identical or identical wearable devices can simplify the synchronization because they are likely to function in a highly similar manner for example as much as individual processing speeds are concerned. It is, however, also possible that all wearable devices are different and/or that another amount of wearable devices, for example four wearable devices, is used.

In typical embodiments, the method comprises a first calibration phase, wherein the first calibration phase comprises the steps:

- each wearable device measures a first characteristic point of a body signal, wherein the body signal is typically an ECG signal of the living organism, wherein the characteristic point is typically an R-peak of a QRS complex of the ECG signal;
- each wearable device determines time-stamps of subsequent characteristic points, typically being of a same type as the first characteristic point, based on its particular counter;
- each wearable device sends the time-stamps to the coordination device.

It is absolutely mandatory that each wearable device participates in the first calibration phase. For example, it is possible that one or more wearable devices are located too remotely on a body (for example on the arm or leg) and that a body signal such as an ECG cannot be measured. Therefore, in typical embodiments, only those wearable devices which are actually able to receive an ECG signal at the location of the body where they are placed participate in the first calibration phase. In this case, only those wearable device which participate in the first calibration phase measure a first characteristic point of a body signal and carry out the subsequent steps of the first calibration phase. Furthermore, a body signal in the sense of the present disclosure is not necessarily a direct body signal. In typical embodiments the body signal is, at least for one or more wearable devices, an indirect body signal, for example an electrical impulse sent out by a wearable device able to receive an ECG signal. This concept is explained in more detail further below.

A time-stamp in the sense of the present disclosure can be an absolute time, for example 11:58:34 or 17:44:12, or yet a relative time, like for example 1.24 seconds. The advantage of a calibration phase during which each wearable device sends time-stamps corresponding to characteristic points of a body signal of the body to which the wearable devices are attached to the coordination device is the following: in this way, the coordination device can become aware of synchronization problems between the different wearable devices, for example because it can become aware of the fact that the time-stamps for characteristic points of a body signal - such as the ECG - do not match for all wearable devices and that at least some wearable devices are thus not properly synchronized. In fact, an ECG signal typically propagates so quickly through the body that wearable devices which are configured to detect this ECG signal but which are located at different locations of the body should all receive the ECG signal at essentially the same time (i.e. with a negligible time shift in the range of a few microseconds), and thus all time-stamps should theoretically be the same for all wearable devices if the devices are properly synchronized. If they are not, this hints towards a synchronization problem, and the coordination device can in such a case take appropriate measures, for example re-initiating a synchronization. The advantage of the transmission of several time-stamps is that it makes it easier for the coordination device to for example detect a systematic "drag behind" of one or more wearable devices. However, in theory one time-stamp per wearable device can be enough for the coordination device to detect a synchronization problem that needs connection.

At this point, one reason why good synchronization is desirable when using various wearable devices for measuring body functions shall be pointed out: one possible way to use the measured body function data is to display the body functions on a screen, for example a screen of the coordination device, with a common time axis. If the synchronization between the wearable devices is not good, then the representation on the screen is improper because the data from the different wearable devices is misaligned on the time axis.

In typical embodiments, the first calibration phase is followed by a second calibration phase, wherein the second calibration phase comprises:

- a time offset calculation step during which the coordination device calculates a particular time offset value for at least one of the wearable devices, preferably for each wearable device, based on the time-stamps received from the wearable devices,
- an offset transmission step during which the coordination device transmits at least to each of those wearable devices for which the particular time offset value does not equal "0" and/or to each of those wearable devices for which a time offset value has been calculated its particular time offset value;
- a time adjustment step during which each wearable device that received its particular time offset value from the coordination device uses this particular time offset value to offset its particular wearable device time.

The inventors have found that it can sometimes be tricky to reach good synchronization because the transmission of the application time information from the coordination device, the processing time of this application time information in the different wearable devices as well as the time until the wearable devices start their respective counters based on the application time information are not necessarily equal across the multitude of wearable devices. With the proposed second calibration step, each wearable device that is not properly synchronized receives a customized time offset value and can thus adjust its particular wearable device time based on this offset value. Like this, the overall synchronization of the multitude of wearable devices can be improved. However, such a second calibration step is not absolutely mandatory. It would for example also be possible that the coordination device simply re-initiates the synchronization process as often as necessary until any time shifts between the different wearable devices are in an acceptable range. Furthermore, it would also be possible to not at all tell the wearable devices about their possible respective mis-synchronization and to only keep track of them in the coordination device. However, in such a case, it would be more difficult to switch the coordination device subsequently. In other words: It will be easier to switch the coordination device later on if the wearable device times of all wearable devices are properly synchronized.

In typical embodiments, during the time offset calculation step, the coordination device calculates a mean time difference based on the time stamps for at least one of the wearable devices and saves this mean time difference as the particular time offset value. The calculation of a mean time difference is advantageous because it can equal out variations and can therefore lead to more reliable time offset values. The mean time difference can correspond to an arithmetic mean or to a median.

In typical embodiments, the time offset calculation step comprises the sub-steps:

- the coordination device preferably chooses one wearable device as reference wearable device;
- the coordination device calculates time differences between the time-stamps of the reference wearable device and corresponding time-stamps of each other wearable device such as to obtain a multitude of time differences for each pair of the reference wearable device and any of the other wearable devices;
- for each pair of the reference wearable device and any of the other wearable devices, the coordination device calculates a mean time difference based on the respective multitude of time differences;
- for each other wearable device, the coordination device saves the determined mean time difference as the particular time offset value;

and the offset transmission step comprises the sub-step:

- the coordination device transmits to each of the other wearable devices its particular time offset value.

This particular configuration of the time offset calculation step has the advantage to lead to comparably precise time offset values for all wearable devices and therefore to a comparably precise synchronization result.

In typical embodiments, the wearable devices only transmit information to the coordination device when the signal quality is good enough. In this context, the expression "information" relates in particular to time-stamps and/or waveforms and/or body signals. The advantage of this is for example that the synchronization method is optimized because the coordination device is not bothered with noisy information that could have a negative impact on the calculations carried out by the coordination device, for example the calculations of the time offset values. In particular embodiments, a wearable device that is experiencing noise sends a noise flag to the coordination device when noise is present and/or sends a noise start flag to the coordination device when noise starts and a noise end flag when noise has ended. In typical embodiments, autocorrelation is used to identify appropriate time offset values in cases where noise is present. In typical embodiments, autocorrelation is at least partly performed by shifting body function signals from at least two wearable devices against each other on a time axis until an optimal correlation is found.

In typical embodiments, the characteristic point is a peak of pulse or a foot of pulse and/or not all wearable devices use the same type of characteristic point, wherein each wearable device preferably uses a different type of characteristic point. The use of pulse wave characteristics as characteristic points has the advantage of making it possible to enable synchronization also in cases when ECG measurements are not available. The combination of different types of characteristic points, for example R-peaks and peaks of pulse has the advantage of allowing synchronization also in cases where some but not all wearable devices can measure ECG signals. In embodiments where different types of characteristic points are combined, a constant offset is typically applied to the time-stamps relating to one or more of the different characteristic points.

In typical embodiments, a characteristic point, in particular an R-peak, is considered to be noisy if an RMS of an accelerometer value around that particular R-peak exceeds a certain threshold. This is advantageous because the wearable devices often comprise accelerometers in any case, because the RMS calculation is straightforward and because the higher the RMS of an accelerometer value is the more noise is typically comprised in the body signals, in particular the ECG, measured by the particular wearable device. RMS is an abbreviation for "Root Mean Square".

At least one wearable device periodically sends a distinct shape electrical impulse, preferably a square shape impulse, through the body to the other wearable devices so that the other wearable devices can use that distinct shape electrical impulse for synchronization. This has for example the advantage that wearable devices which are not able to measure an ECG signal - for example because they are located too far away from the heart, for example at a wrist - can use this distinct shape electrical impulse as characteristic point and can establish their time-stamps to be sent to the coordination device based on these electrical impulses. In typical embodiments, the distinct shape electrical impulse is in line with an ECG signal, typically with an R-peak of the ECG signal. In typical embodiments, the distinct shape electrical impulse is used as indirect body signal by at least some of the wearable devices during the first calibration phase and/or during the second calibration phase.

The distinct shape electrical impulse has an amplitude that is higher than a typical amplitude of an ECG signal, wherein the amplitude of the distinct shape electrical impulse is preferably higher than 10 mV, more preferably higher than 15 mV, most preferably higher than 20 mV.

In typical embodiments, the distinct shape electrical impulse is generated by at least one wearable device when this at least one wearable device measures a characteristic point on a particular body signal, such as the R-wave of the ECG signal or the foot and/or peak of the pulse signal. This has the advantage of allowing a good periodicity of the distinct shape electrical impulse.

In typical embodiments, a moving time window and/or an instantaneous frequency waveform is used for synchronization in heavy noise.

In typical embodiments, at least one timing value is calculated once all patches are synchronized. In typical embodiments, the timing value comprises a Pulse Arrival Time (PAT) and/or a Pre-Ejection Period (PEP) and/or a Pulse Wave Velocity (PWV).

## FIGURES

In the following, the invention is described in detail by means of drawings, wherein show

Figure 1: a schematic view of a part of a human body to which two wearable devices are attached (one wearable device attached to chest, one wearable device attached to arm),

Figure 2: a schematic diagram of an ECG signal and an associated pulse signal,

Figure 3: a schematic view of a part of a human body to which two wearable devices are attached (both wearable devices attached to arm),

Figure 4: a schematic diagram of an ECG signal and an associated electrical impulse signal.

## Description of Preferred Embodiments

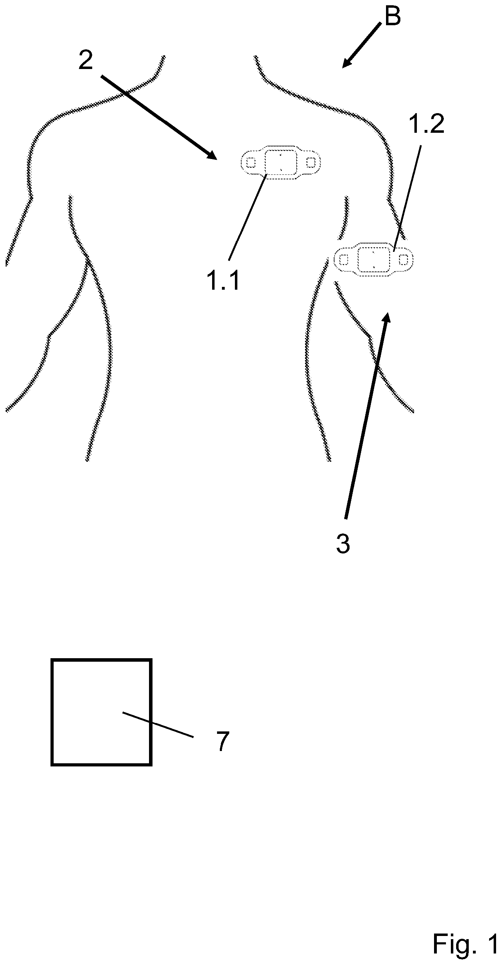

Figure 1 shows a part of a human body B. Two wearable devices 1.1, 1.2 are attached to the human body B. The wearable devices 1.1, 1.2 have the form of wearable patches, which are attached to the human body B by means of an adhesive surface. Each of the wearable patches 1.1, 1.2 comprises an electronic circuitry, wherein the electronic circuitry comprises a processor and a multitude of sensors, especially a pulse sensor, an ECG sensor and an accelerometer, and wherein the electronic circuitry comprises a receiver and a transmitter. Furthermore, each wearable patch 1.1, 1.2 comprises an adhesive surface by means of which they are attached to the body B.

Wearable patch 1.1 is attached to a chest 2 of the human body B and wearable patch 1.2 is attached to an upper arm 3 of the human body B.

The two wearable patches 1.1, 1.2 are time synchronized by means of a coordination device 7. The coordination device 7, which can for example be a mobile phone, has a software application running on it. This software application enables and/or facilitates connection between the coordination device 7 and the wearable patches 1.1, 1.2, transmission and reception of data to/from the wearable devices 1.1, 1.2 as well as visualization of this data, for example data relating to body functions of the human body B sampled by each wearable patch 1.1, 1.2.

When a user places a wearable patch 1.1, 1.2 on the body B (for example on a chest 2, as shown for wearable patch 1.21 in Figure 1) and connects the wearable patch 1.1, 1.2 to the software application running on the coordination device 7, the software application transmits an application time information to this particular wearable patch 1.1, 1.2. In the case where the coordination device 7 is a mobile phone, this application time information is typically the current time of the mobile phone.

In preferred embodiments, the connection protocol used for the communication between the coordination device 7 and the wearable patches 1.1, 1.2 is Bluetooth or Wireless or any standard protocol. In preferred embodiments, the current time of the mobile phone is a network time (such as provided by the mobile carrier network) or a time set by the user.

In the wearable patch 1.1, 1.2, a processor stores the application time information and starts a counter for maintaining a wearable device time. This is preferably done by using a sampling rate of one of the sensors of the wearable patch 1.1, 1.2. For example, if the pulse sensor of a particular wearable patch 1.1, 1.2 is sampled at 250 Hz, then after receiving 250 samples from the pulse sensor, the wearable device time on the processor of that wearable patch 1.1, 1.2 is updated by 1 second. To have a finer granularity, for each sample received from the sensor, the timer can be updated by 1/sampling rate value. For example, where the sampling rate of a sensor is 250 Hz, for each sample received from the sensor, the wearable device time is updated by 4 milliseconds.

When a second wearable patch 1.1, 1.2 is placed on the human body B (for example on an arm 3, as shown for wearable patch 1.2 in Figure 1), it is also connected to the same software application running on the coordination device 7. Just as the first wearable patch 1.1. 1.2, this second wearable patch 1.1, 1.2 also receives the application time information from the software application, stores the application time information in its processor and starts a counter for maintaining a wearable device time. The same typically happens for any further wearable devices placed on the human body B (even if Figure 1 only shows two wearable patches 1.1, 1.2). Thus, for each of the different wearable patches 1.1, 1.2 (and any additional wearable devices) placed on different locations of the human body B, a reference time based on the current time of the coordination device 7 is set.

In other preferred embodiments, the coordination device 7 comprises or is a Bluetooth hub, a computer or a router.

In other preferred embodiments, another protocol to connect the wearable devices 1.1, 1.2 to the coordination device 7 is used, for example any another wireless protocol, such as Zigbee, WiFi or GSM. It is also possible to combine different protocols.

Due to the nature of the Bluetooth protocol or any other underlying protocol used for connecting the coordination device 7 to the wearable devices, a slight delay between transmission of the application time information from the coordination device 7 to the wearable devices on one hand and the actual receipt and storage of the application time information on the processor of the wearable devices on the other hand can occur. This delay can be in the order of milliseconds to few seconds, depending on the mechanism of the underlying protocol and distance between the coordination device 7 and the wearable devices. This delay is therefore not necessarily constant for all wearable devices.

In a preferred embodiment, the method for synchronization of a multitude of wearable devices therefore comprises a first calibration phase for improving the time synchronization of the different wearable devices placed on a body. During this first calibration phase, each wearable device measures a characteristic point of the ECG signal, such as the R peak of the QRS complex. Each wearable device then transmits time-stamps of the subsequent R peaks to the coordination device.

In a preferred embodiment, the method for synchronization of a multitude of wearable devices furthermore comprises a second calibration phase for further improving the time synchronization of the different wearable devices placed on a body. During this second calibration phase, the differences between the time stamps of the R peaks from the different wearable devices are calculated in the coordination device. The mean or median value of multiple differences of the time stamps can be calculated. In the case shown in Figure 1 (i.e. where there are two wearable devices 1.1, 1.2), the coordination device 7 chooses the time stamp from one of the wearable devices 1.1, 1.2 to be reference and transmits the time difference to the other wearable device 1.1, 1.2. The other wearable device 1.1, 1.2 adjusts its particular wearable device time based on the difference. After this step, the second calibration phase is completed. The body signals from the two wearable devices 1.1, 1.2 can now be displayed on the same screen with the same time reference. The second calibration phase can of course also be carried out with more than two wearable devices.

During the first calibration phase and the second calibration phase, the ECG is thus used for calibration. The ECG is an electrical signal that propagates quickly (in microseconds) across the body, as the body is a good conductor of electricity. The pulse wave, on the other hand, could take hundreds of milliseconds to move from the heart to a peripheral part of the body. Thus, ECG acquired from distinct points of the body can be used for improving synchronization of the timing information of the corresponding wearable devices.

In preferred embodiments, the time synchronization is performed only when the data acquisition of the wearable devices is of good quality. For example, for each patch, the time differences between two subsequent R peaks (the RR interval) are computed on the wearable device when the signal quality is good, and are transmitted to the coordination device with the time stamp of the R-peak and/or the second R-peak. In case of a bad signal quality, the R peaks are discarded from computation. The values of RR intervals from the different patches are stored on the coordination device. After a predefined time, the autocorrelation of the RR intervals from the different wearable devices at different time lags are performed. The time lag that provides the best correlation is then transmitted to one of the wearable devices (or several of the wearable devices, in case there are more than two wearable devices) for time synchronization.

It is also possible to perform the described synchronization method using pulse signals, with peak or foot of the pulse used as a characteristic point (similar to the R peak of ECG). The same synchronization mechanism can also be applied between the ECG on one patch and pulse on the other patch. However, as the pulse signal usually takes some time to reach a point in the body, a constant offset needs to be added to the time difference in such a case.

In preferred embodiments, the RMS (Root Mean Square) of the accelerometer value delivered by an accelerometer of a particular wearable device is checked around the R peak, and if the RMS exceeds a threshold, the R-peak value and timing information are not transmitted by this particular wearable device to the coordination device and the signal is marked as noisy.

In another aspect of the invention, once all wearable devices are synchronized, certain timing values between the body signals measured by the different wearable devices are computed.

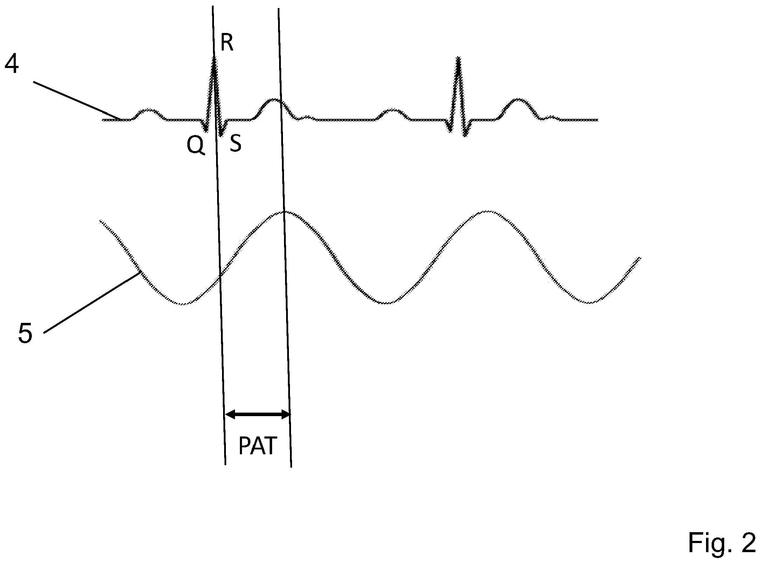

For example, the Pulse Arrival Time (PAT) is such a timing value. An example of an ECG signal 4 and pulse signal 5 with the PAT marked is shown in Figure 2. The PAT is computed as the time interval between the ECG R-peak and a characteristic point on the pulse signal (such as the pulse peak or pulse foot - in Figure 2, it is shown for the pulse peak). The pulse arrival time has two components, Pre-Ejection Period (PEP) and the time the pulse wave takes from the blood flow out of aorta to the point at which the patch is placed. The distance between the blood flow out of aorta and the point where the patch is placed is represented by k (in meters). The PAT is computed using the following formula:

$$PAT = PEP + k/PWV,$$

wherein PWV is the pulse wave velocity.

When two wearable patches are placed on the same user at different points, the PAT at the two points can be used to compute PWV and PEP values. Let k1 and k2 be the distances from the heart to the point where two patches are placed. Then the respective PAT can be computed as follows:

$$PAT1 = PEP + k1/PWV$$

$$PAT2 = PEP + k2/PWV$$

The PWV is computed using the following equation:

$$PWV = (k1 - k2)/(PAT1-PAT2)$$

Once the PWV is computed, the PEP value can be computed. The precise determination of the timing values PAT, PEP and PWV is advantageous because these timing values can give valuable insights into possible malfunctions of the body. It is important to understand that the better the wearable devices placed on the body which are used to calculate these timing values are synchronized, the more precisely the timing values can be determined and the more precisely body malfunctions can be detected.

One of the challenges of the method for computing the timing values described above is that the distances k1, k2 etc. of where the patches are placed from the heart need to be computed. This can be challenging with differing geometries of the arteries for each user.

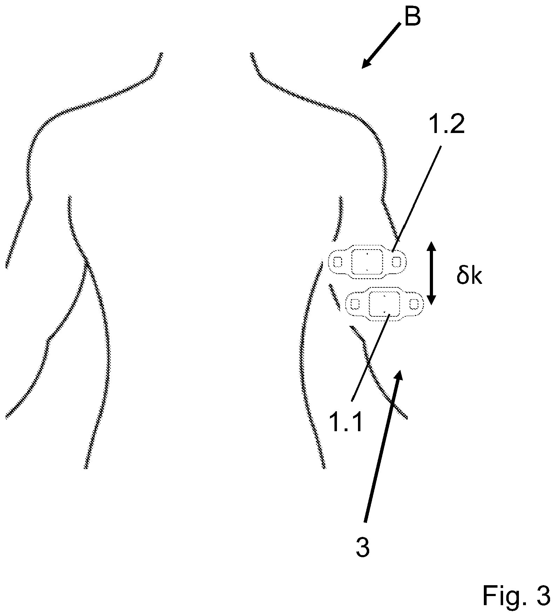

In another aspect of the invention, in order to yet improve the determination of the timing values, two wearable patches 1.1, 1.2 are placed at a predetermined distance δk from each other. For example, the patches could be placed next to each other, horizontally stacked on an upper arm 3 of a human body B, as shown in Figure 3.

In this case, the PWV is computed as follows:

$$PWV = δk/(PAT1-PAT2),$$

where δk is the distance between the wearable patches 1.1, 1.2.

In another aspect of the invention, two wearable patches are put on two sides of a body of a living organism. For example, one wearable patch is put on each of the upper arms of a human body, and both wearable patches are placed at the same height. In an experimental phase, the PWV is calculated by another method, such as placing two wearable patches at a known location or using a different technique, such as the arterial tonometer. The height of the user is also taken as input for the method. The differences in the pulse arrival times (PAT1 and PAT2) are computed from the patches on both sides, along with the height of the user and are correlated with the PWV computed by the other method. This correlation is further used to build a model of (PAT1-PAT2) and height with measured PWV. In actual usage, this model is used to measure PWV, for a given (PAT1-PAT2) and a given height of the user.

In yet another embodiment of the invention, two wearable patches are placed on chest and abdomen and synchronization according to the invention is performed using the ECG R-peaks or pulse wave. The pulse signals of the two wearable patches are further aligned and filtered by a low pass filter with the respiration band. The two signals are added, and a new signal is created. This signal is used as respiration wave of the user.

Even though the determination of the timing values is explained for wearable patches in the above, the determination of the timing values is of course in principle possible with any case of wearable device.

In preferred embodiments, each wearable device, in particular each wearable patch comprises an additional circuitry for inducing an electric current, preferably a low amplitude current, into the body to which the wearable devices are attached at a predetermined rate, preferably mimicking an electric potential of the Sino Arterial node of the heart. In typical embodiments, the shape of the injected current, which is also referred to as signal, pulse, impulse or electrical impulse signal, is fixed to a value distinct from that of the ECG ORS complex (for example a square wave instead of a triangular QRS complex). When a wearable device injects such a signal into the body of the user of the wearable device, other wearable devices attached to the same body typically receive the signal in the same channel as they would use for sampling the ECG signal. Since the shape of the induced signal is distinct, it can be separated from the ECG signal of the heart.

In a typical embodiment, the wearable device injecting the electrical impulse signal does so every time it receives an R-peak from the ECG signal. This electrical impulse signal is received quasi-instantaneously at the other wearable devices attached to the same body (due to low resistance of the human body).

The wearable devices which receive the electrical impulse signal can either discard it (typically if they receive the ECG signal themselves) or can use it as an indirect indication of the R-peak of the ECG signal. In particular, in typical embodiments, wearable devices which are placed at parts of the body where they cannot measure the ECG signal (e.g. on an arm or a leg) receive the electrical impulse signal and use it as indirect body signal for use in the first calibration phase.

In typical embodiments, the injected electrical impulse signal is set to have a completely different amplitude (for example much higher) than the ECG signal of the heart, so that the receiving wearable devices can easily differentiate the injected electrical impulse signal from the heart ECG signal.

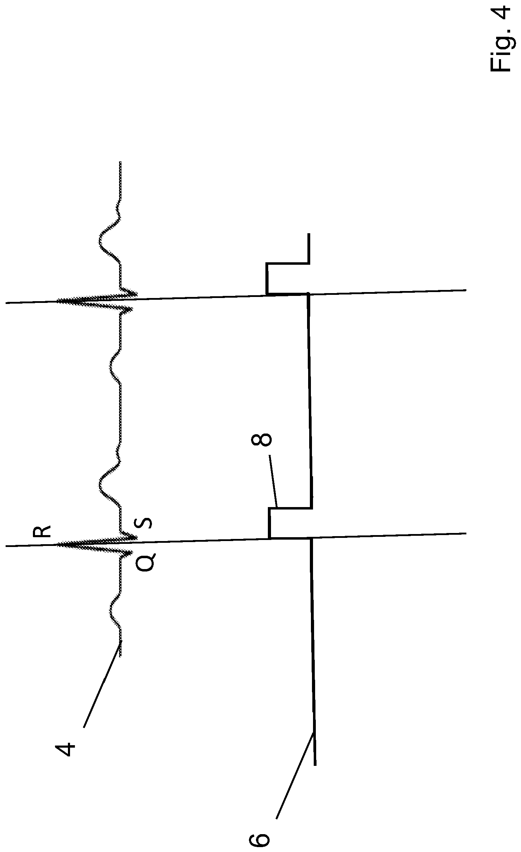

Figure 4 shows an example of an ECG signal 4 and an associated electrical impulse signal 6. Typically, a wearable device which monitors a body's ECG signal 4 creates the electrical impulse signal 6 by forming a rectangular electrical impulse 8 at every time it determines an R-peak in the ECG signal 4. This wearable device injects the electrical impulse signal 6 into the body and thereby enables other wearable devices to make use of the electrical impulse signal 6, for example by using it as an indirect body signal during the first calibration phase.

For example, in the situation shown in Figure 1, one could imagine that the wearable patch 1.1 attached to the chest 2 is able to measure the ECG signal of the human body B but that the wearable patch 1.2 attached to the arm 3 is not able to receive the ECG signal of the human body B. If the wearable patch 1.1 injects the electrical impulse signal 6 into the human body B, then the wearable patch 1.2 can use the electrical impulse signal 6 as (indirect) body signal and thereby participate in the first calibration phase and typically also in the second calibration phase. This has the advantage to achieve better synchronization results because also remotely located wearable devices can participate in the first and second calibration phases.

In certain embodiments, particular steps are taken for synchronization in heavy noise. For example, the ECG and pulse signals can be highly corrupted by movement of the user or other noise sources, such as 50 Hz/60 Hz power supply noise, electromagnetic interference, etc. In particular embodiments, an adaptive filter is used to carry out the timing synchronization under heavy noise. For this, the ECG signal for a pre-determined time window is stored on a wearable device, e.g. a wearable patch. The time window is moved in a sliding fashion, so that computations can be performed in a finer granularity of time window. In particular embodiments, the ECG signal for eight seconds is stored on the wearable device for analysis, and the sliding window has a width of one second, so each second computations are performed using the preceding eight seconds of the ECG signal. The noise on the ECG signal is adaptive cancelled using the accelerometer data of the wearable device in question in the same interval.

In typical embodiments, after the noise cancellation, an adaptive band-pass filter is used to track the instantaneous frequency component, which is associated with the heart rate of the user. The instantaneous frequency waveform over a pre-determined time interval is then sent to the coordination device. In particular embodiments, the instantaneous frequency is computed every one second on an ECG data of eight seconds, in a sliding window fashion. The frequency computed every second for a 60-second duration is then sent to the coordination device.

In typical embodiments, the auto correlation of the frequency signals from two wearable devices at different time stamps is performed on the coordination device, and the time lag that maximizes the correlation is chosen as the time difference between the time stamps on the wearable devices and used for synchronization.

In the above described method, if the signal is noisy, multiple instantaneous frequencies could appear over each time window. In such a case, in one typical embodiment of the invention, the maximum frequency across the different ones is chosen for the window. In another aspect of the invention, the frequency component that is closest to the frequency component chosen in the previous window (or over several previous windows) is chosen as the value.

The invention is applicable to wearable devices, electronic skin sensors for patient monitoring at homes, hospitals, as well as healthy subject monitoring. In fact, the method of synchronization can be used for synchronizing watches or other devices with one another on the same user, using the user's bio signals. The invention is not limited to the preferred embodiments described here. The scope of protection is defined by the claims.

It is further to be noted that methods disclosed in the specification or in the claims may be implemented by a device having means for performing each of the respective acts of these methods.

**Reference list**

| 1.1, 1.2 | Wearable device / Wearable patch |
|---|---|
| 2 | Chest |
| 3 | Upper arm |
| 4 | ECG signal |
| 5 | Pulse signal |
| 6 | Electrical impulse signal |
| 7 | Coordination device |
| 8 | Rectangular electrical impulse |

| B | Human body |
|---|---|

**Claims**

1. Method for synchronization of a multitude of wearable devices (1.1, 1.2),

   - wherein each wearable device (1.1, 1.2) is attached to a body (B) of a living organism,

wherein

- a software application running on a coordination device (7) sends an application time information to each wearable device (1.1, 1.2) such as to allow the wearable devices (1.1, 1.2) to synchronize,

- each wearable device (1.1, 1.2) stores the application time information and starts a counter for maintaining a wearable device time in line with the application time information, wherein

- in each particular wearable device (1.1, 1.2), the counter of that particular wearable device (1.1, 1.2) uses a sampling rate of at least one pulse sensor comprised in that particular wearable device (1.1, 1.2) for counting, **characterized in that**

- at least one wearable device (1.1, 1.2) periodically sends a distinct shape electrical impulse (8) through the body to the other wearable devices (1.1, 1.2) so that the other wearable devices (1.1, 1.2) can use that distinct shape electrical impulse (8) for synchronization, wherein

- the distinct shape electrical impulse (8) has an amplitude that is higher than a typical amplitude of an ECG signal (4).

2. Method according to claim 1, **characterized in that** at least one of the wearable devices (1.1, 1.2) is a wearable patch (1.1, 1.2) for monitoring at least one body signal (4, 5, 6), in particular a pulse signal (5) and/or an ECG signal (4), of the body (B).

3. Method according to any of the preceding claims, **characterized in that** the multitude of wearable devices (1.1, 1.2) comprises at least two, preferably at least three, more preferably at least five wearable devices (1.1, 1.2), wherein the wearable devices (1.1, 1.2) are preferably essentially identical.

4. Method according to any of the preceding claims, **characterized in that** the method comprises a first calibration phase, wherein the first calibration phase comprises the steps:

- each wearable device (1.1, 1.2) measures a first characteristic point of a body signal (4, 5, 6), wherein the body signal (4, 5, 6) is typically an ECG signal (4) of the living organism, wherein the characteristic point is typically an R-peak of a QRS complex of the ECG signal;

- each wearable device (1.1, 1.2) determines time-stamps of subsequent characteristic points, typically being of a same type as the first characteristic point, based on its particular counter;

- each wearable device (1.1, 1.2) sends the time-stamps to the coordination device (7).

5. Method according to claim 4, **characterized in that** the first calibration phase is followed by a second calibration phase, wherein the second calibration phase comprises:

- a time offset calculation step during which the coordination device (7) calculates a particular time offset value for at least one of the wearable devices (1.1, 1.2), preferably for each wearable device (1.1, 1.2), based on the time-stamps received from the wearable devices (1.1, 1.2),

- an offset transmission step during which the coordination device (7) transmits at least to each of those wearable devices (1.1, 1.2) for which the particular time offset value does not equal "0" and/or to each of those wearable devices (1.1, 1.2) for which a time offset value has been calculated its particular time offset value;

- a time adjustment step during which each wearable device (1.1, 1.2) that received its particular time offset value from the coordination device (7) uses this particular time offset value to offset its particular wearable device time.

6. Method according to claim 5, **characterized in that** during the time offset calculation step, the coordination device (7) calculates a mean time difference based on the time stamps for at least one of the wearable devices (1.1, 1.2) and saves this mean time difference as the particular time offset value.

7. Method according to any of the claims 5 or 6, **characterized in that**

- the time offset calculation step comprises the sub-steps:

- the coordination device (7) preferably chooses one wearable device (1.1, 1.2) as reference wearable device (1.1, 1.2);

- the coordination device calculates time differences between the time-stamps of the reference wearable device (1.1, 1.2) and corresponding time-stamps of each other wearable device (1.1, 1.2) such as to obtain a multitude of time differences for each pair of the reference wearable device (1.1, 1.2) and any of the other wearable devices (1.1, 1.2);

- for each pair of the reference wearable device (1.1, 1.2) and any of the other wearable devices (1.1, 1.2), the coordination device (7) calculates a mean time difference based on the respective multitude of time differences;

- for each other wearable device (1.1, 1.2), the coordination device (7) saves the determined mean time difference as the particular time offset value, and

- the offset transmission step comprises the substep:

  - the coordination device (7) transmits to each of the other wearable devices (1.1, 1.2) its particular time offset value.

8. Method according to any of the preceding claims, **characterized in that** the wearable devices (1.1, 1.2) only transmit information to the coordination device (7) when the signal quality is good enough.

9. Method according to any of the claims 4 to 8, **characterized in that** the characteristic point is a peak of pulse or a foot of pulse and/or that not all wearable devices (1.1, 1.2) use the same type of characteristic point, wherein each wearable device (1.1, 1.2) preferably uses a different type of characteristic point.

10. Method according to any of the claims 4 to 9, **characterized in that** a characteristic point, in particular an R-peak, is considered to be noisy if an RMS of an accelerometer value around that particular R-peak exceeds a certain threshold.

11. Method according to any of the preceding claims, **characterized in that** the distinct shape electrical impulse (8) is a square shape impulse.

12. Method according to claim 11, **characterized in that** the amplitude of the distinct shape electrical impulse (8) is higher than 10 mV, more preferably higher than 15 mV, most preferably higher than 20 mV.

13. Method according to any of the claims 11 to 12, **characterized in that** the distinct shape electrical impulse (8) is generated by at least one wearable device (1.1, 1.2) when this at least one wearable device (1.1, 1.2) measures a characteristic point on a particular body signal (4, 5), such as the R-wave of the ECG signal (4) or the foot and/or peak of the pulse signal (5).

**Patentansprüche**

1. Verfahren zur Synchronisation einer Mehrzahl von tragbaren Geräten (1.1, 1.2),

   - wobei jedes tragbare Gerät (1.1, 1.2) an einem Körper (B) eines lebenden Organismus befestigt ist,
   wobei
   - eine Softwareanwendung, die auf einem Koordinationsgerät (7) läuft, eine Anwendungszeitinformation an jedes tragbare Gerät (1.1, 1.2) sendet, um es den tragbaren Geräten (1.1, 1.2) zu erlauben, sich zu synchronisieren,
   - jedes tragbare Gerät (1.1, 1.2) die Anwendungszeitinformation speichert und einen Zähler startet, um eine Tragbargerät-Zeit mit der Anwendungszeitinformation im Einklang zu halten, wobei
   - in jedem einzelnen tragbaren Gerät (1.1, 1.2) der Zähler dieses einzelnen tragbaren Geräts (1.1, 1.2) eine Abtastrate von mindestens einem Pulssensor verwendet, der in diesem bestimmten tragbaren Gerät (1.1, 1.2) zum Zählen enthalten ist,
   **dadurch gekennzeichnet, dass**
   - mindestens ein tragbares Gerät (1.1, 1.2) periodisch einen eindeutigen elektrischen Impuls (8) durch den Körper zu den anderen tragbaren Geräten (1.1, 1.2) sendet, so dass die anderen tragbaren Geräte (1.1, 1.2) diesen eindeutigen elektrischen Impuls (8) für die Synchronisation verwenden können, wobei
   - der eindeutige elektrische Impuls (8) eine Amplitude hat, die grösser als eine typische Amplitude eines EKG-Signals (4) ist.

2. Verfahren nach Anspruch 1, **dadurch gekennzeichnet, dass** mindestens eines der tragbaren Geräte (1.1, 1.2) ein tragbarer Patch (1.1, 1.2) zur Überwachung mindestens eines Körpersignals (4, 5, 6), insbesondere eines Pulssignals (5) und/oder eines EKG-Signals (4), des Körpers (B) ist.

3. Verfahren nach einem der vorhergehenden Ansprüche, **dadurch gekennzeichnet, dass** die Mehrzahl an tragbaren Geräten (1.1, 1.2) mindestens zwei, bevorzugt mindestens drei, bevorzugter mindestens fünf tragbare Geräte (1.1, 1.2) umfasst, wobei die tragbaren Geräte (1.1, 1.2) vorzugsweise im Wesentlichen identisch sind.

4. Verfahren nach einem der vorhergehenden Ansprüche, **dadurch gekennzeichnet, dass** das Verfahren eine erste Kalibrierungsphase umfasst, wobei die erste Kalibrierungsphase die Schritte umfasst:

   - jedes tragbare Gerät (1.1, 1.2) misst einen ersten charakteristischen Punkt eines Körpersignals (4, 5, 6), wobei das Körpersignal (4, 5, 6) typischerweise ein EKG-Signal (4) des lebenden Organismus ist, wobei der charakteristische Punkt typischerweise ein R-Peak eines QRS-Komplexes des EKG-Signals ist;
   - jedes tragbare Gerät (1.1, 1.2) bestimmt Zeitstempel von nachfolgenden charakteristischen Punkten, die typischerweise von einem gleichen Typ wie der erste charakteristische Punkt sind, basierend auf seinem speziellen Zähler;
   - jedes tragbare Gerät (1.1, 1.2) sendet die Zeitstempel an das Koordinationsgerät (7).

5. Verfahren nach Anspruch 4, **dadurch gekennzeichnet, dass** auf die erste Kalibrierungsphase eine zweite Kalibrierungsphase folgt, wobei die zweite Kalibrierungsphase umfasst:

- einen Zeitversatzberechnungsschritt, während dessen das Koordinationsgerät (7) einen bestimmten Zeitversatzwert für mindestens eines der tragbaren Geräte (1.1, 1.2), vorzugsweise für jedes tragbare Gerät (1.1, 1.2), basierend auf den von den tragbaren Geräten (1.1, 1.2) empfangenen Zeitstempeln, berechnet,

- einen Versatzübertragungsschritt, während dessen das Koordinationsgerät (7) mindestens zu jedem dieser tragbaren Geräte (1.1, 1.2), für die der bestimmte Zeitversatzwert nicht gleich "0" ist, und/oder zu jedem dieser tragbaren Geräte (1.1, 1.2), für die ein Zeitversatzwert berechnet wurde, seinen bestimmten Zeitversatzwert überträgt;

- einen Zeitanpassungsschritt, während dessen jedes tragbare Gerät (1.1, 1.2), das seinen bestimmten Zeitversatzwert von dem Koordinationsgerät (7) empfangen hat, diesen bestimmten Zeitversatzwert verwendet, um seine bestimmte Tragbargerät-Zeit auszugleichen.

6. Verfahren nach Anspruch 5, **dadurch gekennzeichnet, dass** während des Zeitversatzberechnungsschritts das Koordinationsgerät (7) eine mittlere Zeitdifferenz basierend auf den Zeitstempeln für mindestens eines der tragbaren Geräte (1.1, 1.2) berechnet und diese mittlere Zeitdifferenz als den bestimmten Zeitversatzwert speichert.

7. Verfahren nach einem der Ansprüche 5 oder 6, **dadurch gekennzeichnet, dass**

- der Zeitversatzberechnungsschritt die Teilschritte umfasst:

- das Koordinationsgerät (7) wählt vorzugsweise ein tragbares Gerät (1.1, 1.2) als Referenz-Tragbargerät (1.1, 1.2);

- das Koordinationsgerät berechnet Zeitdifferenzen zwischen den Zeitstempeln des Referenz-Tragbargeräts (1.1, 1.2) und entsprechenden Zeitstempeln jedes anderen tragbaren Geräts (1.1, 1.2), um eine Mehrzahl von Zeitdifferenzen für jedes Paar aus Referenz-Tragbargerät (1.1, 1.2) und einem der anderen tragbaren Geräte (1.1, 1.2) zu erhalten;

- für jedes Paar aus Referenz-Tragbargerät (1.1, 1.2) und einem der anderen tragbaren Geräte (1.1, 1.2) berechnet das Koordinationsgerät (7) eine mittlere Zeitdifferenz basierend auf der jeweiligen Mehrzahl von Zeitdifferenzen;

- für jedes andere tragbare Gerät (1.1, 1.2) speichert das Koordinationsgerät (7) die ermittelte mittlere Zeitdifferenz als den bestimmten Zeitversatzwert, und

- der Versatzübertragungsschritt den Teilschritt umfasst:

- das Koordinationsgerät (7) überträgt an jedes der anderen tragbaren Geräte (1.1, 1.2) seinen jeweiligen Zeitversatzwert.

8. Verfahren nach einem der vorhergehenden Ansprüche, **dadurch gekennzeichnet, dass** die tragbaren Geräte (1.1, 1.2) nur dann Informationen an das Koordinationsgerät (7) übertragen, wenn die Signalqualität gut genug ist.

9. Verfahren nach einem der Ansprüche 4 bis 8, **dadurch gekennzeichnet, dass** der charakteristische Punkt eine Pulsspitze oder ein Pulsfuss ist und/oder dass nicht alle tragbaren Geräte (1.1, 1.2) dieselbe Artvon charakteristischem Punkt verwenden, wobei jedes tragbare Gerät (1.1, 1.2) vorzugsweise eine andere Art von charakteristischem Punkt verwendet.

10. Verfahren nach einem der Ansprüche 4 bis 9, **dadurch gekennzeichnet, dass** ein charakteristischer Punkt, insbesondere ein R-Peak, als verrauscht angesehen wird, wenn ein RMS eines Beschleunigungsmessgerätwerts um diesen bestimmten R-Peak einen bestimmten Schwellenwert überschreitet.

11. Verfahren nach einem der vorhergehenden Ansprüche, **dadurch gekennzeichnet, dass** der eindeutige elektrische Impuls (8) ein Rechteckimpuls ist.

12. Verfahren nach Anspruch 11, **dadurch gekennzeichnet, dass** die Amplitude des eindeutigen elektrischen Impulses (8) grösser als 10 mV, bevorzugter grösser als 15 mV, am meisten bevorzugt grösser als 20 mV ist.

13. Verfahren nach einem der Ansprüche 11 bis 12, **dadurch gekennzeichnet, dass** der eindeutige elektrische Impuls (8) von mindestens einem tragbaren Gerät (1.1, 1.2) erzeugt wird, wenn dieses mindestens eine tragbare Gerät (1.1, 1.2) einen charakteristischen Punkt auf einem bestimmten Körpersignal (4, 5), wie die R-Welle des EKG-Signals (4) oder den Fuss und/oder die Spitze des Pulssignals (5), misst.

## Revendications

1. Procédé de synchronisation d'une multitude de dispositifs portables (1.1, 1.2),

   - dans lequel chaque dispositif portable (1.1, 1.2) est attaché au corps (B) d'un organisme vivant, dans lequel
   - une application logicielle exécutée sur un dispositif de coordination (7) envoie une information de temps d'application à chaque dispositif portable (1.1, 1.2) de manière à permettre aux dispositifs portables (1.1, 1.2) de se synchroniser,
   - chaque dispositif portable (1.1, 1.2) stocke l'information de temps d'application et démarre un compteur pour maintenir un temps de dispositif portable en phase avec l'information de temps d'application, dans lequel
   - dans chaque dispositif portable particulier (1.1, 1.2), le compteur de ce dispositif portable particulier (1.1, 1.2) utilise un taux d'échantillonnage d'au moins un capteur de pouls compris dans ce dispositif portable particulier (1,1, 1,2) pour compter,
   **caractérisé en ce que**
   - au moins un dispositif portable (1.1, 1.2) envoie périodiquement une impulsion électrique de forme distincte (8) à travers le corps aux autres dispositifs portables (1.1, 1.2) de telle sorte que les autres dispositifs portables (1.1, 1.2) peuvent utiliser cette impulsion électrique de forme distincte (8) pour synchronisation, dans lequel
   - l'impulsion électrique de forme distincte (8) a une amplitude qui est supérieure à une amplitude typique d'un signal ECG (4).

2. Procédé selon la revendication 1, **caractérisé en ce qu'**au moins un des dispositifs portables (1.1, 1.2) est un patch portable (1.1, 1.2) pour surveiller au moins un signal corporel (4, 5, 6), en particulier un signal de pouls (5) et/ou un signal ECG (4), du corps (B).

3. Procédé selon l'une quelconque des revendications précédentes, **caractérisé en ce que** la multitude de dispositifs portables (1.1, 1.2) comprend au moins deux, de préférence au moins trois, plus préférablement au moins cinq dispositifs portables (1.1, 1.2), dans lequel les dispositifs portables (1.1, 1.2) sont de préférence essentiellement identiques.

4. Procédé selon l'une quelconque des revendications précédentes, **caractérisé en ce que** le procédé comprend une première phase d'étalonnage, où la première phase d'étalonnage comprend les étapes :

   - chaque dispositif portable (1.1, 1.2) mesure un premier point caractéristique d'un signal corporel (4, 5, 6), où le signal corporel (4, 5, 6) est typiquement un signal ECG (4) de l'organisme vivant, où le point caractéristique est typiquement un pic R d'un complexe QRS du signal ECG;
   - chaque dispositif portable (1.1, 1.2) détermine des horodatages de points caractéristiques ultérieurs, typiquement d'un même type que le premier point caractéristique, sur la base de son compteur particulier ;
   - chaque dispositif portable (1.1, 1.2) envoie les horodatages au dispositif de coordination (7).

5. Procédé selon la revendication 4, **caractérisé en ce que** la première phase d'étalonnage est suivie d'une seconde phase d'étalonnage, où la seconde phase d'étalonnage comprend :

   - une étape de calcul de décalage temporel au cours de laquelle le dispositif de coordination (7) calcule une valeur de décalage temporel particulière pour au moins l'un des dispositifs portables (1.1, 1.2), de préférence pour chaque dispositif portable (1.1, 1.2), sur la base des horodatages reçus en provenance des dispositifs portables (1.1, 1.2),
   - une étape de transmission du décalage au cours de laquelle le dispositif de coordination (7) transmet au moins à chacun de ces dispositifs portables (1.1, 1.2) pour lesquels la valeur de décalage temporel particulière n'est pas égale à « 0 » et/ou à chacun de ces dispositifs portables (1.1, 1.2) pour lesquels une valeur de décalage temporel a été calculée sa valeur de décalage temporel particulière;
   - une étape d'ajustement temporel au cours de laquelle chaque dispositif portable (1.1, 1.2) qui a reçu sa valeur de décalage temporel particulière en provenance du dispositif de coordination (7) utilise cette valeur de décalage temporel particulière pour décaler son temps de dispositif portable particulier.

6. Procédé selon la revendication 5, **caractérisé en ce que** pendant l'étape de calcul de décalage temporel, le dispositif de coordination (7) calcule une différence de temps moyenne sur la base des horodatages pour au moins l'un des dispositifs portables (1.1, 1.2) et sauvegarde cette différence de temps moyenne en tant que valeur de décalage temporel particulière.

7. Procédé selon l'une quelconque des revendications 5 ou 6, **caractérisé en ce que**

   - l'étape de calcul de décalage temporel comprend les sous-étapes :

- le dispositif de coordination (7) choisit de préférence un dispositif portable (1.1, 1.2) en tant que dispositif portable de référence (1.1, 1.2) ;

- le dispositif de coordination calcule des différences de temps entre les horodatages du dispositif portable de référence (1.1, 1.2) et des horodatages correspondants de chaque autre dispositif portable (1.1, 1.2) de façon à obtenir une multitude de différences de temps pour chaque paire du dispositif portable de référence (1.1, 1.2) et l'un quelconque des autres dispositifs portables (1.1, 1.2);

- pour chaque paire du dispositif portable de référence (1.1, 1.2) et l'un quelconque des autres dispositifs portables (1.1, 1.2), le dispositif de coordination (7) calcule une différence de temps moyenne sur la base de la multitude respective des différences de temps;

- pour chaque autre dispositif portable (1.1, 1.2), le dispositif de coordination (7) sauvegarde la différence de temps moyenne déterminée en tant que valeur de décalage temporel particulière, et

- l'étape de transmission de décalage comprend la sous-étape :

- le dispositif de coordination (7) transmet à chacun des autres dispositifs portables (1.1, 1.2) sa valeur de décalage temporel particulière.

8. Procédé selon l'une quelconque des revendications précédentes, **caractérisé en ce que** les dispositifs portables (1.1, 1.2) transmettent uniquement des informations au dispositif de coordination (7) lorsque la qualité du signal est suffisamment bonne.

9. Procédé selon l'une quelconque des revendications 4 à 8, **caractérisé en ce que** le point caractéristique est un pic de pouls ou un pied de pouls et/ou que les dispositifs portables (1,1, 1,2) n'utilisent pas tous le même type de point caractéristique, où chaque dispositif portable (1.1, 1.2) utilise de préférence un type différent de point caractéristique.

10. Procédé selon l'une quelconque des revendications 4 à 9, **caractérisé en ce qu'**un point caractéristique, en particulier un pic R, est considéré comme bruyant si un RMS d'une valeur d'accéléromètre autour de ce pic R particulier dépasse un certain seuil.

11. Procédé selon l'une quelconque des revendications précédentes, **caractérisé en ce que** l'impulsion électrique de forme distincte (8) est une impulsion de forme carrée.

12. Procédé selon la revendication 11, **caractérisé en ce que** l'amplitude de l'impulsion électrique de forme distincte (8) est supérieure à 10 mV, plus préférablement supérieure à 15 mV, le plus préférablement supérieure à 20 mV.

13. Procédé selon l'une quelconque des revendications 11 à 12, **caractérisé en ce que** l'impulsion électrique de forme distincte (8) est générée par au moins un dispositif portable (1.1, 1.2) lorsque cet au moins un dispositif portable (1.1, 1.2) mesure un point caractéristique sur un signal de corps particulier (4, 5), tel que l'onde R du signal ECG (4) ou le pied et/ou le pic du signal de pouls (5).

Fig. 1

Fig. 2

Fig. 3

4

R

Q  S

6

8

Fig. 4

**EP 3 836 828 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2018013569 A1
- US 2014275888 A1